# EUROPEAN PATENT APPLICATION

(11) **EP 3 272 367 A1**
(43) Date of publication of application: **24.01.2018**
(21) Application number: 17181553.3
(22) Date of filing: 14.07.2017
(51) Int. Cl.: A61L 9/12, B65D 25/10

(54) **FRAGRANCE CONTAINER**

(30) Priority: 20.07.2016 CN 201620775716 U
(71) Applicant: Lam, Chan Va, Tianjin (CN)
(72) Inventor: Lam, Chan Va, Tianjin (CN)
(74) Representative: Bailey, David Martin

(57) **Abstract**

The present invention relates to a novel container of scented sheet comprising a body (20) and at least one slot (10), with the size of the opening of the slot (10) gradually reducing from outside to inside. The tapering, fading or reducing design for the slot, i.e., the size of the opening gradually reduced from outside to inside, which is not only convenient for the storing, but also can ensure the stability after the scented sheet package is installed.

## Description

### Technical field

The present invention relates to a novel fragrance container, especially a container in the form of a scented pouch or sheet.

### The prior art

In particular, the present invention is concerned with fragrancing containers for air-freshener devices, especially those including a fan or heater to aid dispersion of the fragrance. In such devices, the fragrance is often formed as a gel or as an open container. Thus the scented sheet or container is not well isolated or sealed and the fragrance is prone to volatilize, normally at an inconsistent or irregular rate. Thus the stability of the scented sheet cannot be guaranteed after the latter is put inside.

### The contents of the invention

The technical problem solved by the present invention is to provide a novel container of scented sheet in respect to the defects in the prior art.

To solve the technical problem, the technical solution employed by the present invention is to form a novel container of scented sheet comprising a body and at least one slot, with the size or width of an opening of the slot gradually reducing from outside to inside.

In the novel container of scented sheet, many air holes are provided at the rear end of said body (20).

In the novel container of scented sheet, a lock slot is provided at the side of said body.

The novel container of scented sheet according to the present invention is implemented with the following beneficial effects: The invention adopts the fading design for the slot, i.e., the size of the opening gradually reduced from outside to inside, which is not only convenient for the storing, but also can ensure the stability after the scented sheet packet is installed.

### Description of the Figure

The above and other aspects of the present invention will now be described in further detail, by way of example only, with reference to the accompanying figures..
Fig.1 is a structural schematic from a first direction of an embodiment of an embodiment of a container of fragranced or scented sheet or pouch according to the present invention;

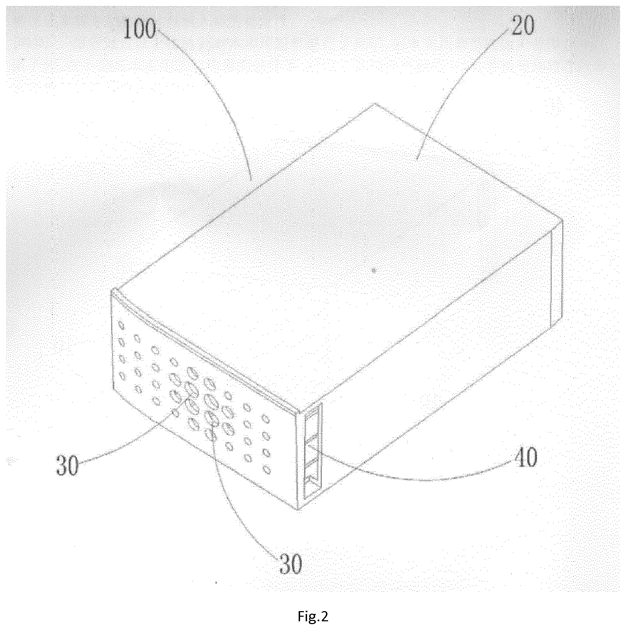

Fig.2 is a structural schematic of a second direction container of Fig.1.

### The detailed embodiment

In order to clarify the purpose, technical solution and advantage of the present invention, a detailed description of the present invention will be given by embodiment in connection with the Figures. It should be understood that, the detailed embodiment described herein is merely for explanation, but not limitation of the present invention.

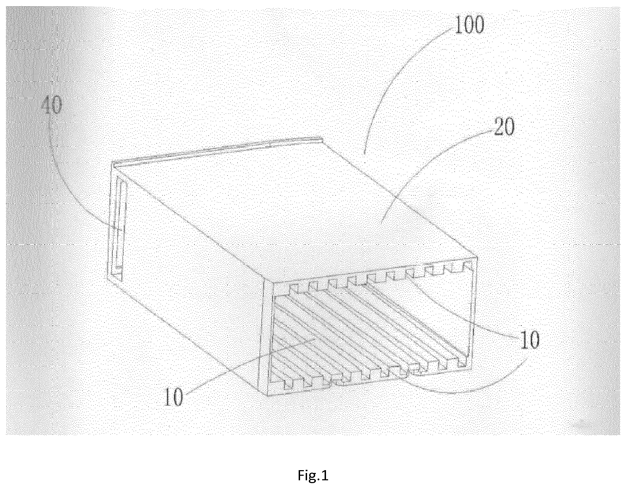

Fig.1 shows a container of scented sheet, comprising a body 20 and at least one slot 10, formed as opposed channels between opposed inner faces of the body 20, with the width or size of the opening of the slot 10 gradually reduces or tapers from an outside to an inside face, which is not only convenient for the storage, but also can ensure the stability after the scented sheet packet is installed.

Many air holes 30 are provided at the rear end of said body 20, to facilitate the emission of the odor of the scented sheet. A lock slot 40 is provided at the side of said body 20 to interact with a corresponding feature in the body in a fragrancing device such as a device having a battery-powered fan, to retain the body 20 in the device. In the embodiment shown, the optimum number of slots 10 is at least five, preferably about nine. However, there can be any number of slots.

The main function of the slot of the scented sheet package is to store the scented sheet. The procedure of using it is as follows: Toggle off the lock at a side of the fragrancing device containing the container of the present invention, the container will automatically pop up, the scented sheet package is pushed into the container along the slot manually, pushing against the taper of the slot to retain the scented sheet package in the slot and then the container is pushed back into the body of the device.

The invention adopts the tapering design for the slot, i.e., the size of the opening gradually reduced from outside to inside, which is not only convenient for the storing, but also can ensure the stability after the scented sheet package is installed.

Although the present invention is disclosed by virtue of embodiment above, the protective scope of the present invention is not limited thereto. Without departing from the spirit of the present invention, any modification and substitute to each part above will fall into the claimed scope of the present invention.

In providing, in preferred embodiments, containers for a plurality of scented sheet packages, the present invention allows the user to vary the concentration of the fragrance delivered by simply increasing the number of packages inserted. Additionally, by providing the scented packages in a range of fragrances, the user can select different combinations of fragrances, to create a personalized fragrance combination.

## Claims

1. A container of scented sheet, **characterized in that** the container of scented sheet (100) comprises a body (20) and at least one slot (10), with the size of the opening of the slot (10) gradually reduced from outside to inside.

2. A container for scented or fragranced sheets or pouches, the container having at least one slot defined by opposing channels in opposing internal faces of the container, wherein the at least one slot tapers or gradually reduces in width from an insertion face of the container to a near end or face of the container.

3. A container of scented sheet, according to claim 1 or claim 2, wherein many air holes (30) are provided at the rear end of said body (20).

4. A container of scented sheet, according to any preceding claim, wherein a lock slot (40) is provided at the side of said body (20).

5. A container as claimed in any preceding claim comprising a plurality of slots, preferably about 10 slots.
